# FASCICULE DE BREVET EUROPEEN

(11) **EP 3 490 631 B1**
(45) Date de publication et mention de la délivrance du brevet: **24.02.2021**
(21) Numéro de dépôt: 17754411.1
(22) Date de dépôt: 26.07.2017
(51) Int. Cl.: A61M 1/10, A61M 1/12

(54) **PROGRAMME ET UNITÉ DE GESTION D'UNE POMPE CARDIAQUE**
PROGRAM UND EINHEIT ZUR STEUERUNG EINER HERZPUMPE
PROGRAM AND CONTROL UNIT FOR MANAGING A CARDIAC PUMP

(30) Priorité: 26.07.2016 FR 1657188
(43) Date de publication de la demande: 05.06.2019
(73) Titulaire: Fineheart, 33600 Pessac (FR)
(72) Inventeur: GARRIGUE, Stéphane, 33130 Begles (FR); MASCARELL, Arnaud, 37250 Montbazon (FR)
(74) Mandataire: Plasseraud IP
(86) Numéro de dépôt international: PCT/FR2017/052093
(87) Numéro de publication internationale: WO 2018/020161

(56) Documents cités:
- US-A- 6 066 086
- US-A1- 2008 133 006
- US-A1- 2014 323 796
- US-A1- 2016 206 797

## Description

### ARRIERE-PLAN DE L'INVENTION

### Domaine de l'invention

La présente invention concerne un programme d'ordinateur comprenant des instructions adaptées à la mise en oeuvre d'un procédé de gestion d'une pompe cardiaque implantable, laquelle est destinée à assister un ventricule affaibli d'un individu, durant la phase systolique, et une unité de gestion permettant de mettre en oeuvre le procédé.

### Arrière-plan technologique

L'insuffisance cardiaque (IC), est un état pathologique dans lequel le cœur d'un patient présente une incapacité à fournir un débit sanguin nécessaire aux besoins métaboliques de l'organisme.

Il est connu pour traiter l'insuffisance cardiaque d'implanter un dispositif d'assistance ventriculaire (DAV), qui est une pompe cardiaque artificielle.

Cette pompe mécanique ne remplace pas le cœur qui continue à fonctionner, mais apporte une aide au ventricule affaibli afin d'accroître le débit sanguin de façon adaptée aux besoins de l'individu.

Cette assistance peut être temporaire dans l'attente d'un greffon disponible pour réaliser une transplantation cardiaque.

Cependant, on observe une proportion significative de patients qui ne recevront pas un tel greffon, soit parce qu'ils ne peuvent être candidats à une telle transplantation, par exemple en raison d'une insuffisance cardiaque sévère, soit parce qu'aucun greffon adapté n'est disponible pour ces patients.

Dans ce cas, l'assistance ventriculaire est utilisée en destination, c'est-à-dire que la pompe cardiaque artificielle est implantée à long terme.

Ces pompes cardiaques font donc l'objet d'intenses recherches visant à améliorer la survie et la qualité de vie des patients présentant une insuffisance cardiaque.

De nombreuses avancées ont été réalisées ces dernières années et on connaît aujourd'hui des dispositifs d'assistance ventriculaire plus compacts, silencieux et présentant une durée de service accrue.

Les pompes cardiaques implantables de l'état de l'art sont ainsi typiquement équipées d'un moteur électrique intégré pour assurer leur fonctionnement, la vitesse de rotation de la pompe fournissant la force nécessaire pour faire circuler le sang depuis le ventricule affaibli vers la circulation corporelle (voir p.ex. US 2008/0133006).

Le contrôleur et la source d'alimentation de la pompe cardiaque sont typiquement placés à l'extérieur du patient. Une ligne percutanée au niveau de l'abdomen assure alors la liaison entre la pompe fixée à la paroi du ventricule et ces éléments externes.

Bien que représentant un progrès certain pour la qualité de vie d'un patient souffrant d'insuffisance cardiaque, de nombreux inconvénients sont encore constatés.

D'abord, le patient est limité dans sa mobilité par la présence d'un boitier externe auquel il est relié en permanence. Les douches et baignades ne sont pas toujours autorisées. Et surtout, des risques d'infection sont susceptibles de survenir au niveau de l'orifice de passage de la ligne percutanée.

On cherche donc à ce que la pompe cardiaque soit la plus autonome possible et implantée avec son contrôleur et sa source d'alimentation.

L'individu atteint d'une insuffisance cardiaque retrouvera ainsi son intégrité corporelle et gagnera en mobilité.

La demande de brevet WO2013014339 A1 au nom de la présente demanderesse décrit une pompe cardiaque électrique particulièrement fiable et aisée d'installation dont l'unité de contrôle et sa batterie électrique sont implantées.

Toutefois, on observe que les pompes les plus récentes fonctionnent sur un rythme sensiblement constant sans ajustement réel aux besoins instantanés du patient et à son activité physique.

En outre, l'autonomie de la batterie implantée est moindre qu'une batterie extérieure ce qui implique des recharges plus régulières de celle-ci.

Bien que cette recharge puisse se faire éventuellement par transduction percutanée, elle constitue une contrainte récurrente et pénible pour le patient.

Il existe donc un besoin pressant pour une méthode de réglage d'une pompe cardiaque implantée permettant d'optimiser sa consommation électrique afin d'augmenter l'intervalle de temps entre deux recharges.

### Objet de l'invention

La présente invention vise à pallier les inconvénients de l'art antérieur et à répondre aux contraintes ci-dessus énoncées en proposant un programme d'ordinateur selon la revendication 1, comprenant des instructions adaptées à la mise en oeuvre d'un procédé de gestion d'une pompe cardiaque implantable, simple dans sa conception et dans son mode opératoire, fiable et permettant d'ajuster le réglage de cette pompe sur le battement naturel du cœur d'un individu.

Un autre objet de la présente invention est un tel programme qui permette à la pompe cardiaque de répondre en temps réel aux efforts physiques de cet individu.

Encore un objet de cette invention est un tel programme autorisant un fonctionnement de la pompe cardiaque en mode continu en cas de défaillance du cœur de cet individu.

La présente invention vise également une unité de gestion implantable selon la revendication 2 comprenant une unité centrale et une source d'alimentation d'une pompe cardiaque, cette unité centrale comprenant un logiciel permettant de régler cette pompe sur le fonctionnement naturel du cœur de l'individu.

### BREVE DESCRIPTION DE L'INVENTION

A cet effet, cette divulgation concerne un procédé de gestion (non revendiqué) d'une pompe cardiaque destinée à assister le cœur d'un patient, ladite pompe cardiaque étant destinée à envoyer du sang sous pression à un débit proportionnel à la vitesse de rotation Vᵣₚₘ de ladite pompe au travers de la valve aortique dudit cœur.

On réalise, pendant une même systole ventriculaire, les étapes suivantes :
(a) détecter la fermeture de la valve mitrale dudit cœur, la vitesse de rotation Vᵣₚₘ de ladite pompe étant strictement inférieure à une valeur maximale V_{rpm max} de vitesse de rotation de ladite pompe,
(b) augmenter la vitesse de rotation Vᵣₚₘ de ladite pompe de sorte qu'à un instant t₂, postérieur à l'instant t₁ correspondant à la fermeture de ladite valve mitrale, la vitesse de rotation de ladite pompe est égale, ou sensiblement égale, à ladite valeur maximale V_{rpm max} de vitesse de rotation,
(c) maintenir ladite vitesse de rotation Vᵣₚₘ de ladite pompe à cette valeur maximale V_{rpm max} pendant au moins une partie de la période de temps T pendant laquelle la valve aortique est ouverte, et
répéter éventuellement les étapes (a) à (c) pour au moins la systole ventriculaire d'un cycle cardiaque suivant.

De manière avantageuse, ce procédé de gestion autorise ainsi un réglage "physiologique" de la pompe cardiaque, c'est-à-dire un réglage au plus proche du fonctionnement naturel du cœur du patient.

La pompe cardiaque n'atteint sa valeur maximale de vitesse de rotation que pour fournir la force nécessaire à l'éjection du sang au travers de l'aorte et reste, de préférence, à une valeur minimale en dehors de cette phase afin de maintenir une vitesse de rotation de la pompe constante et de limiter sa consommation électrique. A l'étape (a), on cherche donc à déterminer le début de la période de contraction iso-volumétrique, la pompe cardiaque artificielle n'étant pas à sa valeur maximale de vitesse de rotation mais à une valeur strictement inférieure, et encore mieux à sa valeur minimale de vitesse de rotation.

A l'étape (c), on maintient la vitesse de rotation Vᵣₚₘ de la pompe à une valeur égale ou sensiblement égale à la valeur maximale V_{rpm max} de vitesse de rotation de la pompe. Durant cette période de temps T, la vitesse de rotation Vᵣₚₘ de la pompe est, par conséquent, constante, ou sensiblement constante.

Bien entendu, après l'étape c), on réalise de préférence une étape d) supplémentaire dans laquelle on diminue la vitesse de rotation de la pompe de sorte que la vitesse de rotation Vᵣₚₘ de ladite pompe est inférieure strictement à la valeur maximale V_{rpm max} de la vitesse de rotation de ladite pompe.

Une telle pompe cardiaque est généralement implantée dans le ventricule dédié à la circulation sanguine pour alimenter le corps du patient en oxygène au travers de la valve aortique. Ce rôle est normalement dévolu au ventricule gauche. Il se peut dans de rares cas que ce soit le ventricule droit qui assure cette fonction. Pour cette raison, on entendra ici par "valve mitrale", la valve d'entrée du ventricule, c'est-à-dire la valve auriculo-ventriculaire. De même, on entendra par "valve aortique", la valve de sortie du ventricule, c'est-à-dire la valve sigmoïde.

Dans différents modes de réalisation particuliers de ce procédé, chacun ayant ses avantages particuliers et susceptibles de nombreuses combinaisons techniques possibles:
- à un instant t₃ correspondant à la fermeture, ou sensiblement à la fermeture, de la valve aortique, on réalise une étape supplémentaire (d) consistant à diminuer la vitesse de rotation Vᵣₚₘ de ladite pompe à une valeur strictement inférieure à la valeur maximale V_{rpm max} de vitesse de rotation de ladite pompe.

De manière avantageuse, la vitesse de rotation Vᵣₚₘ de la pompe cardiaque à la fin de l'étape (d) est égale, ou sensiblement égale, à la vitesse de rotation Vᵣₚₘ de ladite pompe à l'étape (a).

De préférence, cette pompe cardiaque est réglée de sorte que sa vitesse de rotation Vᵣₚₘ est égale, ou sensiblement égale, à une valeur de consigne en dehors des étapes b) à d). Avantageusement, cette valeur de consigne correspond à une valeur minimale V_{rpm min} de vitesse de rotation de ladite pompe.

Cette vitesse V_{rpm min} minimale est alors déterminée de manière à maintenir le sang présent dans le ventricule en mouvement et empêcher la formation de caillots sanguins tout en minimisant la consommation électrique du moteur de la pompe cardiaque.

Les valeurs maximale V_{rpm max} et/ou minimale V_{rpm min} de vitesse de rotation de la pompe sont de préférence limitées à des valeurs pré-définies et ajustables. La valeur maximale V_{rpm max} de vitesse de rotation n'est ainsi pas nécessairement égale à la vitesse de rotation maximale que peut atteindre cette pompe cardiaque. Au contraire, cette valeur maximale V_{rpm max} peut dépendre de paramètres physiologiques du patient et être alors un pourcentage de cette vitesse de rotation maximale de la pompe.
- préalablement à l'étape (a), on mesure l'activité électrique du cœur pour détecter une dépolarisation ventriculaire dudit cœur et en ce qu'à partir de l'instant t₀ correspondant à la détection de ladite dépolarisation, on réalise de manière successive lesdites étapes a) à c).

Ainsi, la mesure de la dépolarisation du cœur permet avantageusement de synchroniser les différentes étapes du procédé sur l'activité cardiaque réel du cœur du patient. A partir de cet instant t₀ correspondant à la détection de la dépolarisation ventriculaire, on ouvre une fenêtre de détection ayant une durée prédéfinie pour la détection de la fermeture de la valve mitrale. Cette détection est réalisée de préférence par la mesure des vibrations mécaniques audibles et/ou des vibrations mécaniques inaudibles liées à l'activité mécanique du cœur. La mesure de ces vibrations mécaniques permet de générer des signaux qui sont ensuite analysés en vue de détecter le ou les signaux liés à la fermeture de la valve mitrale. Cette mesure est réalisée au moyen d'un ou plusieurs capteurs de vibrations choisis parmi un microphone, un accéléromètre et des combinaisons de ces éléments.

De préférence, la mesure de ces vibrations liées à l'activité mécanique du cœur est réalisée à un instant t'₀ = t₀ + t_{blanking} postérieur à l'instant t₀. La durée de temporisation t'₀ - t₀ est réglable et déterminée de manière à minimiser ou éliminer des bruits parasites liés à l'activité mécanique du cœur afin de faciliter la détection de la fermeture de la valve mitrale.

Avantageusement, la mesure de l'activité électrique du cœur est réalisée au moyen d'au moins une électrode ventriculaire. Cette électrode est en contact avec la paroi ventriculaire. Elle peut être placée sur la surface intérieure ou extérieure de ladite paroi ventriculaire.
- l'étape de détection de la fermeture de la valve mitrale est réalisée au moyen d'au moins un accéléromètre implantable.

La mise en œuvre d'un ou plusieurs accéléromètres implantables permet de détecter les vibrations mécaniques inaudibles liées à l'activité mécanique du cœur. Cet accéléromètre est de préférence placé à l'intérieur du ventricule au plus près de la valve mitrale pour déterminer avec une grande précision et fiabilité l'instant.
- à l'étape (a) et/ou à l'étape (d), on varie progressivement la vitesse de rotation Vᵣₚₘ de ladite pompe.

La pompe cardiaque comportant un moteur électrique alimenté en énergie par une source d'alimentation telle qu'une batterie rechargeable et implantable, une augmentation progressive de la vitesse de rotation de la pompe pour amener celle-ci d'une valeur minimale Vrpm min à une valeur maximale Vrpm max de vitesse de rotation permet avantageusement de minimiser la consommation d'énergie et par conséquent, d'augmenter la durée d'utilisation de la source d'alimentation avant une recharge.
- l'ouverture de la valve aortique dudit cœur survenant physiologiquement à un instant t_{physio}, postérieur à l'instant t₁, la durée Δt séparant les instants t₁ et t₂ est déterminée, à l'étape (b), de sorte que ladite valeur maximale V_{rpm max} de vitesse de rotation de ladite pompe est atteinte avant, à, ou encore après l'instant t_{physio}. Avantageusement, la vitesse de rotation Vᵣₚₘ de ladite pompe étant atteint avant l'instant t_{physio}, on provoque une ouverture précoce de la valve aortique du cœur.

De préférence, cette durée Δt est égale à, ou est sensiblement égale à, la durée de la phase de contraction iso-volumétrique.
- ayant préalablement déterminé la durée de la phase de contraction iso-volumétrique pour ledit patient par échographie ou par imagerie par résonance magnétique (IRM) ou encore par tomographie par émission de positrons, on prend, à l'étape (b), une durée Δt égale à, ou sensiblement égale à, cette durée de la phase de contraction iso-volumétrique.

On simplifie ainsi le procédé en limitant le nombre de mesures nécessaires à sa réalisation.
- la valeur maximale V_{rpm max} de vitesse de rotation de ladite pompe étant ajustable, on varie cette valeur maximale V_{rpm max} pour un patient donné en fonction de son rythme cardiaque et/ou de la contenance du ventricule correspondant.

Ainsi lorsque le rythme cardiaque du patient augmente, cette valeur maximale V_{rpm max} augmente également. De même, pour un volume de sang accru dans le ventricule suite par exemple à un temps de repos additionnel, on augmente la valeur maximale V_{rpm max} de vitesse de rotation de la pompe pour éjecter plus de sang au travers de la valve aortique.
- on contrôle et on régule la vitesse de rotation de ladite pompe cardiaque.

Avantageusement, cette régulation de la vitesse de rotation de la pompe est réalisée, notamment lors de l'étape (b) et/ou l'étape (d), pour éviter l'apparition de pics dans la vitesse de rotation de la pompe. La régulation de la vitesse de rotation de cette pompe peut s'effectuer suivant une logique, soit en boucle ouverte (en mesurant et programmant la fin de la systole (fermeture de la valve aortique)), soit en boucle fermée grâce à la mise en œuvre d'un accéléromètre capable de détecter le bruit de fermeture mitrale mais également le bruit de fermeture aortique signant la fin de la systole ventriculaire.
- mesurant l'activité électrique du cœur, et ayant déterminé à partir de cette mesure un trouble du rythme cardiaque, on réalise les étapes a) à c) une fois toutes les deux systoles ventriculaires consécutives, la vitesse de rotation Vᵣₚₘ de ladite pompe étant maintenue à une valeur minimale V_{rpm min} pendant la systole intermédiaire dite de repos.

Ce maintien de la vitesse de rotation Vᵣₚₘ de la pompe cardiaque à sa valeur minimale V_{rpm min} est réalisé pendant l'intégralité de cette systole intermédiaire, ou encore dite de repos.
- mesurant l'activité électrique du cœur et ayant déterminé à partir de cette mesure que le patient présente une tachycardie ventriculaire ou un arrêt cardiaque, on maintient de manière constante la vitesse de rotation Vᵣₚₘ de la pompe à sa valeur maximale V_{rpm max}, indépendamment des étapes a) à c).
- ladite pompe cardiaque est un dispositif d'assistance ventriculaire (DAV) implantable.

Cette pompe cardiaque est avantageusement ancrée à la paroi du cœur, le patient pouvant dès lors se déplacer de manière active sans aucun risque.

La présente invention concerne un programme d'ordinateur comprenant des instructions adaptées à la mise en œuvre de chacune des étapes du procédé de gestion tel que décrit précédemment, lorsque ce programme est exécuté sur un ordinateur.

Bien entendu, l'expression "programme d'ordinateur" est synonyme des termes "programme" et "logiciel". De même, le terme "ordinateur" s'entend de tout dispositif programmable. Dans un mode de réalisation particulier, ce dispositif programmable est implantable dans le corps du patient et est avantageusement alimenté par une source d'alimentation implantée telle qu'une batterie électrique rechargeable, de sorte que ce dispositif fonctionne de manière autonome pour régler la pompe cardiaque artificielle.

La présente invention concerne encore une unité de gestion implantable. Selon l'invention, cette unité de gestion comprend une source d'alimentation et une unité centrale comportant un processeur, ladite source d'alimentation étant destinée à alimenter en énergie une pompe cardiaque, ladite unité centrale comprenant un ensemble d'instructions logicielles qui lorsqu'elles sont exécutées par ledit processeur, permettent de mettre en œuvre un procédé de gestion de ladite pompe cardiaque, ladite pompe cardiaque étant destinée à envoyer du sang sous pression à un débit proportionnel à la vitesse de rotation Vᵣₚₘ de ladite pompe, ledit procédé comprenant, pendant une même systole ventriculaire, les étapes suivantes :
(a) détecter la fermeture de la valve mitrale dudit cœur, la vitesse de rotation Vᵣₚₘ de ladite pompe étant strictement inférieure à une valeur maximale V_{rpm max} de vitesse de rotation de ladite pompe,
(b) augmenter la vitesse de rotation Vᵣₚₘ de ladite pompe de sorte qu'à un instant t₂, postérieur à l'instant t₁ correspondant à la fermeture de ladite valve mitrale, la vitesse de rotation de ladite pompe est égale, ou sensiblement égale, à ladite valeur maximale V_{rpm max} de vitesse de rotation,
(c) maintenir ladite vitesse de rotation Vᵣₚₘ de ladite pompe à cette valeur maximale V_{rpm max} pendant au moins une partie de la période de temps T pendant laquelle la valve aortique est ouverte, et
répéter éventuellement les étapes (a) à (c) pour au moins la systole ventriculaire d'un cycle cardiaque suivant.

Cette unité de gestion est de préférence reliée à la pompe cardiaque artificielle par une liaison filaire pour alimenter cette pompe et envoyer les signaux de commande de celle-ci.

Elle peut également comprendre un émetteur-récepteur sans fil pour transmettre automatiquement des données telles que des informations sur le rythme cardiaque ou encore l'état de la source d'alimentation implantée, en vue d'un suivi de télémédecine.

La transmission des données peut être réalisée vers un terminal externe portatif au moyen de signaux de communication sans fil à courte portée, par exemple basés sur un protocole bluetooth ou Zigbee, .... Ce terminal externe peut comporter un moyen de communication mettant en œuvre un réseau d'accès cellulaire et/ou un réseau internet pour transmettre ces données vers par exemple un cardiologue. Le réseau d'accès cellulaire peut être de plusieurs types (2G, 3G, 4G), chaque type de réseau étant accessible selon plusieurs technologies d'accès cellulaires (2G : EDGE, GPRS, 3G : UMTS, HSDPA, HSUPA, HSPA, HSPA+, 4G : LTE). Le réseau internet est par exemple un réseau comportant des points d'accès non cellulaires sans fil tel qu'un réseau WLAN, par exemple Wi-Fi ou WiMAX ou encore d'un réseau Li-Fi. Ce terminal externe peut présenter un dispositif d'affichage pour permettre à l'utilisateur de lire des messages ou de choisir des options dans un menu.

De préférence, cette unité centrale comprend une ou plusieurs entrées pour recevoir un ou plusieurs signaux dont chacun est lié à une vibration mécanique audible ou inaudible liée à l'activité mécanique du cœur, ladite unité centrale comprenant un premier sous-ensemble d'instructions logicielles dudit ensemble d'instructions logicielles qui lorsqu'elles sont exécutées par ledit processeur, permettent de définir une fenêtre temporelle de mesure dudit ou desdits signaux, d'analyser chaque signal ainsi reçu à l'entrée de ladite unité centrale durant cette fenêtre temporelle pour déterminer un ou plusieurs paramètres du signal correspondant, de comparer le ou les paramètres de chaque signal ainsi déterminés avec une ou plusieurs données préalablement enregistrées dans une unité de stockage de ladite unité centrale afin d'identifier le signal correspondant à la fermeture de la vanne mitrale ainsi que l'instant t₁ correspondant à la fermeture de ladite valve mitrale.

De manière avantageuse, l'activité électrique du cœur étant mesurée au moyen d'une ou plusieurs électrodes, le ou les signaux de mesure étant reçus à une ou plusieurs autres entrées de ladite unité centrale, ladite unité centrale comprenant un deuxième sous-ensemble d'instructions logicielles dudit ensemble d'instructions logicielles qui lorsqu'elles sont exécutées par ledit processeur, permettent de déterminer en temps réel la fréquence cardiaque du cœur dudit patient, de contrôler ladite pompe cardiaque selon une loi prédéterminée laquelle est fonction de ladite fréquence cardiaque ainsi déterminée, en particulier sa vitesse de rotation Vᵣₚₘ.

De préférence, lesdites instructions logicielles dudit deuxième sous-ensemble permettent également, lorsqu'elles sont exécutées par ledit processeur, de déterminer à partir d'une mesure de l'activité électrique du cœur, chaque instant t₀ auquel se produit une dépolarisation une dépolarisation ventriculaire dudit cœur afin de synchroniser les étapes a) à c) dudit procédé de gestion.

Il est dès lors possible de synchroniser le réglage de la pompe cardiaque sur le rythme naturel du cœur du patient équipé de cette pompe.

### BREVE DESCRIPTION DES DESSINS

D'autres avantages, buts et caractéristiques particulières de la présente invention ressortiront de la description qui va suivre, faite, dans un but explicatif et nullement limitatif, en regard des dessins annexés, dans lesquels :
- la Figure 1 représente schématiquement les différentes étapes du procédé de gestion d'une pompe cardiaque en fonction de l'activité mécanique naturel du cœur d'un individu ;

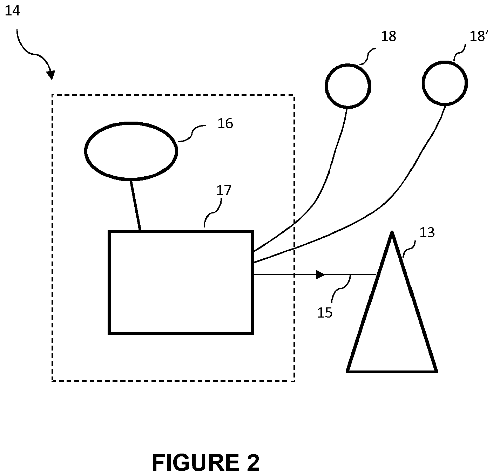

- la Figure 2 représente schématiquement un ensemble de réglage et d'alimentation d'une pompe cardiaque artificielle selon un mode de réalisation particulier de l'invention ;

### DESCRIPTION DETAILLEE DE MODE DE REALISATION DE L'INVENTION

Tout d'abord, on note que les figures ne sont pas à l'échelle.

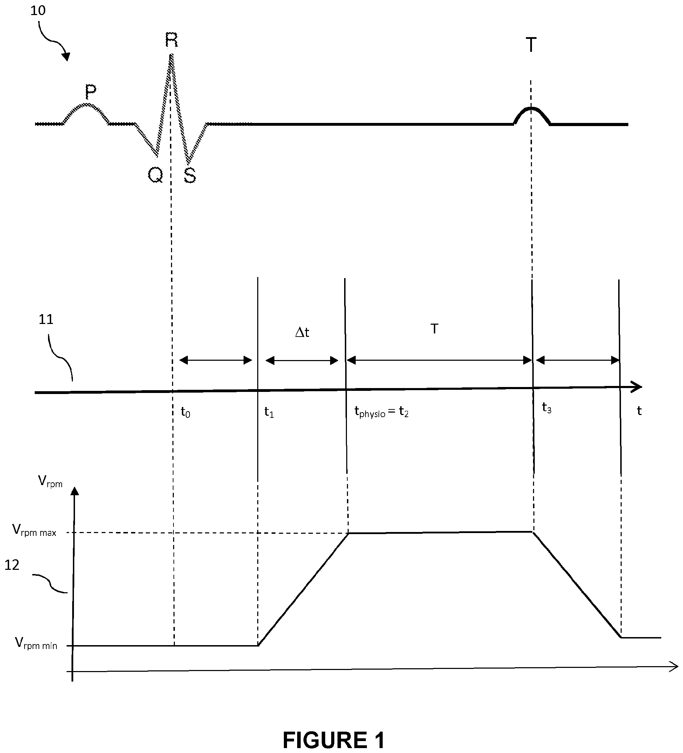

La Figure 1 montre de manière schématique les différentes étapes du procédé de gestion d'une pompe cardiaque en fonction de l'activité mécanique naturel du cœur d'un individu.

Il est connu que la contraction du cœur au repos suit une séquence invariable qu'il est possible de suivre en mesurant l'activité électrique du cœur. On obtient dès lors une courbe 10 de signal électrique en fonction du temps.

La courbe 10 qui représente schématiquement l'activité électrique normale d'un cœur humain, montre essentiellement l'onde P désignant la contraction des oreillettes du cœur, le complexe QRS matérialisant la contraction des ventricules et l'onde T relative à la repolarisation électrique des ventricules.

Cette activité électrique du cœur trouve son corollaire dans l'activité mécanique du cœur qui sera maintenant décrite pour le seul ventricule gauche du cœur au regard d'un axe 11 du temps.

Dans un premier temps, l'oreillette gauche se remplit de sang, la pression y étant supérieure à la pression dans le ventricule gauche. Puis l'oreillette gauche se contracte (onde P) et force le passage du sang vers le ventricule gauche, la valve mitrale s'ouvrant pour libérer ce passage. Après l'intervalle de temps séparant P et R, le ventricule gauche commence à se contracter, la pression augmentant et dépassant la pression dans l'oreillette gauche, la valve mitrale se referme à l'instant t₁.

Cependant la pression dans le ventricule gauche n'est pas encore suffisante pour ouvrir la valve aortique, le volume de la cavité ventriculaire ne changeant pas, on parle de contraction iso-volumétrique.

La pression continue alors de s'élever dans le ventricule gauche jusqu'à ce que la valve aortique s'ouvre à un instant t_{physio}, postérieur à l'instant t₁, pour permettre de chasser le sang contenu dans le ventricule gauche vers la circulation corporelle pendant une phase dite d'éjection.

Cette phase de contraction qui se termine par la fermeture de la valve aortique lorsque la pression dans le ventricule gauche devient inférieure à la pression artérielle s'appelle la systole ventriculaire (intervalle de temps situé au sein de la portion de courbe électrique Q - T sur la courbe 10).

Le cœur est ici pourvu d'une pompe 13 cardiaque permettant d'assister le ventricule gauche affaibli pour projeter le sang depuis le ventricule gauche à travers la valve aortique.

Cette pompe 13 cardiaque est destinée à envoyer du sang sous pression à un débit proportionnel à la vitesse de rotation Vᵣₚₘ de cette pompe 13 au travers de la valve aortique.

Cette pompe 13 artificelle comporte ici un impulseur inséré dans le ventricule gauche à travers la paroi de cœur, une membrane d'étanchéité permettant d'assurer une liaison étanche de l'impulseur et de la paroi du cœur, cette membrane étant en partie suturée sur la paroi externe du cœur, d'un carter solidaire, directement ou indirectement, de la membrane d'étanchéité, ce carter étant placé dans le ventricule gauche et d'un moteur électrique destiné à aspirer et refouler le sang depuis le fond du ventricule gauche.

Une unité 14 de gestion permettant de contrôler cette pompe 13, est reliée à l'impulseur par une liaison filaire 15.

Cette unité 14 de gestion comporte une source 16 d'alimentation électrique pour alimenter en énergie cette pompe 13 ainsi qu'une unité 17 centrale comprenant processeur et une unité de stockage. Cette unité 17 centrale comprend également un ensemble d'instructions logicielles qui lorsqu'elles sont exécutées par ce processeur, permettent de mettre en œuvre un procédé de gestion de cette pompe 13 cardiaque.

Ce procédé de gestion comporte ici une première étape visant à détecter l'instant t₁ correspondant à la fermeture de la valve mitrale, la vitesse de rotation Vᵣₚₘ de ladite pompe 13 étant maintenue à une valeur strictement inférieure à une valeur maximale V_{rpm max} de vitesse de rotation de la pompe 13.

Comme représenté sur la courbe 12, laquelle montre la variation de la vitesse de rotation de la pompe 13 en fonction du temps t, cette valeur de vitesse de rotation est égale à une valeur minimale V_{rpm min} de vitesse de rotation, laquelle est déterminée pour éviter toute stagnation du sang dans le ventricule tout en minimisant la consommation électrique de la pompe 13.

Puis, à partir de cet instant t₁, on augmente la vitesse de rotation Vᵣₚₘ de la pompe 13 de sorte qu'à un instant t₂, postérieur à l'instant t₁, la vitesse de rotation de la pompe 13 est égale, ou sensiblement égale, à sa valeur maximale V_{rpm max} de vitesse de rotation.

De préférence, cette augmentation de la vitesse de rotation de la pompe 13 est progressive pour ne pas solliciter anormalement en énergie la source 16 d'alimentation.

L'instant t₂ est ici choisi de sorte qu'il corresponde à l'ouverture de la valve aortique, la pompe 13 ayant ainsi atteint sa valeur maximale V_{rpm max} de vitesse de rotation pour éjecter le sang au travers de la valve aortique.

Comme représenté sur la Figure 1, la vitesse de rotation de la pompe 13 est alors maintenue constante, et à cette valeur maximale V_{rpm max}, pendant toute la période de temps T pendant laquelle la valve aortique est ouverte de manière à assurer une éjection maximale du sang présent dans le ventricule gauche.

A la fermeture de la valve aortique, c'est-à-dire à l'instant t₃, on réduit progressivement la vitesse de rotation de la pompe 13 cardiaque jusqu'à sa valeur minimale V_{rpm min}.

On répète, de préférence, l'ensemble de ces étapes pour chaque systole ventriculaire suivante de manière à optimiser l'énergie de la source 16 d'alimentation et de réduire le temps entre deux recharges successives de cette source 16 d'alimentation.

Il a été observé que ce procédé apportait un progrès significatif dans la qualité de vie du patient souffrant d'une insuffisance cardiaque.

De préférence, et pour s'adapter automatiquement aux activités physiques du patient, équipé d'une telle pompe 13 cardiaque, on mesure l'activité électrique du cœur de ce patient de sorte à détecter, préalablement à la réalisation de chaque première étape de détection de la fermeture de la valve mitrale, une dépolarisation ventriculaire du cœur du patient.

Cette mesure de dépolarisation est réalisée par le biais d'une ou plusieurs électrodes 18, 18' ventriculaires.

Une telle mesure permet avantageusement de synchroniser les différentes étapes du procédé de gestion par rapport au rythme cardiaque du patient.

En outre, et pour répondre aux besoins métaboliques du patient lors d'un effort, la valeur maximale V_{rpm max} de vitesse de rotation de la pompe est ajustable et peut donc être augmentée pour assurer un débit plus important de sang lorsque nécessaire.

## Revendications

1. Programme d'ordinateur comprenant des instructions adaptées à la mise en œuvre de chacune des étapes du procédé de gestion d'une pompe (13) cardiaque, lorsque ledit programme est exécuté sur un ordinateur, ladite pompe (13) cardiaque étant destinée à envoyer du sang sous pression à un débit proportionnel à la vitesse de rotation Vᵣₚₘ de ladite pompe (13), ledit procédé comprenant, pendant une même systole ventriculaire, les étapes suivantes :
(a) détecter la fermeture de la valve mitrale dudit cœur, la vitesse de rotation Vᵣₚₘ de ladite pompe (13) étant strictement inférieure à une valeur maximale V_{rpm max} de vitesse de rotation de ladite pompe (13),
(b) augmenter la vitesse de rotation Vᵣₚₘ de ladite pompe (13) de sorte qu'à un instant t₂, postérieur à l'instant t₁ correspondant à la fermeture de ladite valve mitrale, la vitesse de rotation de ladite pompe (13) est égale, ou sensiblement égale, à ladite valeur maximale V_{rpm max} de vitesse de rotation,
(c) maintenir ladite vitesse de rotation Vᵣₚₘ de ladite pompe (13) à cette valeur maximale V_{rpm max} pendant au moins une partie de la période de temps T pendant laquelle la valve aortique est ouverte, et
- répéter éventuellement les étapes (a) à (c) pour au moins la systole ventriculaire d'un cycle cardiaque suivant.

2. Unité (14) de gestion implantable **caractérisée en ce qu'**elle comprend une source (16) d'alimentation et une unité (17) centrale comportant un processeur, ladite source (16) d'alimentation étant destinée à alimenter en énergie une pompe (13) cardiaque, ladite unité (17) centrale comprenant un ensemble d'instructions logicielles qui lorsqu'elles sont exécutées par ledit processeur, permettent de mettre en œuvre un procédé de gestion de ladite pompe (13) cardiaque, ladite pompe (13) cardiaque étant destinée à envoyer du sang sous pression à un débit proportionnel à la vitesse de rotation Vᵣₚₘ de ladite pompe (13), ledit procédé comprenant, pendant une même systole ventriculaire, les étapes suivantes :
(a) détecter la fermeture de la valve mitrale dudit cœur, la vitesse de rotation Vᵣₚₘ de ladite pompe (13) étant strictement inférieure à une valeur maximale V_{rpm max} de vitesse de rotation de ladite pompe (13),
(b) augmenter la vitesse de rotation Vᵣₚₘ de ladite pompe (13) de sorte qu'à un instant t₂, postérieur à l'instant t₁ correspondant à la fermeture de ladite valve mitrale, la vitesse de rotation de ladite pompe (13) est égale, ou sensiblement égale, à ladite valeur maximale V_{rpm max} de vitesse de rotation,
(c) maintenir ladite vitesse de rotation Vᵣₚₘ de ladite pompe (13) à cette valeur maximale V_{rpm max} pendant au moins une partie de la période de temps T pendant laquelle la valve aortique est ouverte, et
répéter éventuellement les étapes (a) à (c) pour au moins la systole ventriculaire d'un cycle cardiaque suivant.

3. Unité selon la revendication 2, **caractérisée en ce que** ladite unité (17) centrale comprend une ou plusieurs entrées pour recevoir un ou plusieurs signaux dont chacun est lié à une vibration mécanique audible ou inaudible liée à l'activité mécanique du cœur, ladite unité (17) centrale comprenant un premier sous-ensemble d'instructions logicielles dudit ensemble d'instructions logicielles qui lorsqu'elles sont exécutées par ledit processeur, permettent de définir une fenêtre temporelle de mesure dudit ou desdits signaux, d'analyser chaque signal ainsi reçu à l'entrée de ladite unité (17) centrale durant cette fenêtre temporelle pour déterminer un ou plusieurs paramètres du signal correspondant, de comparer le ou les paramètres de chaque signal ainsi déterminés avec une ou plusieurs données préalablement enregistrées dans une unité de stockage de ladite unité (17) centrale afin d'identifier le signal correspondant à la fermeture de la vanne mitrale ainsi que l'instant t₁ correspondant à la fermeture de ladite valve mitrale.

4. Unité selon la revendication 3, **caractérisée en ce que** l'activité électrique du cœur étant mesurée au moyen d'une ou plusieurs électrodes (18, 18'), le ou les signaux de mesure étant reçus à une ou plusieurs autres entrées de ladite unité (17) centrale, ladite unité (17) centrale comprenant un deuxième sous-ensemble d'instructions logicielles dudit ensemble d'instructions logicielles qui lorsqu'elles sont exécutées par ledit processeur, permettent de déterminer en temps réel la fréquence cardiaque du cœur dudit patient, de contrôler ladite pompe (13) cardiaque selon une loi prédéterminée laquelle est fonction de ladite fréquence cardiaque ainsi déterminée, en particulier sa vitesse de rotation Vᵣₚₘ.

5. Unité selon la revendication 4, **caractérisée en ce que** lesdites instructions logicielles dudit deuxième sous-ensemble permettent également, lorsqu'elles sont exécutées par ledit processeur, de déterminer à partir d'une mesure de l'activité électrique du cœur, chaque instant t₀ auquel se produit une dépolarisation une dépolarisation ventriculaire dudit cœur afin de synchroniser les étapes a) à c) dudit procédé de gestion.

## Patentansprüche

1. Computerprogramm, welches Befehle umfasst, die dazu geeignet sind, jeden der Schritte des Verfahrens zur Steuerung einer Herzpumpe (13) auszuführen, wenn das Programm auf einem Computer ausgeführt wird, wobei die Herzpumpe (13) dazu bestimmt ist, Blut unter Druck mit einem Durchsatz zu fördern, der proportional zur Rotationsgeschwindigkeit Vᵣₚₘ der Pumpe (13) ist, wobei das Verfahren während einer gleichen ventrikulären Systole die folgenden Schritte umfasst:
a) Erfassen des Schließens der Mitralklappe des Herzens, wobei die Drehzahl Vᵣₚₘ der Pumpe (13) grundsätzlich kleiner als ein Maximalwert V_{rpm max} der Drehzahl der Pumpe (13) ist,
b) Erhöhen der Drehzahl Vᵣₚₘ der Pumpe (13), so dass zu einem Zeitpunkt t₂ nach dem Zeitpunkt t₁, der dem Schließen der Mitralklappe entspricht, die Drehzahl der Pumpe (13) gleich oder im Wesentlichen gleich dem Maximalwert V_{rpm max} der Drehzahl ist,
c) Halten der Drehzahl Vᵣₚₘ der Pumpe (13) auf diesem Maximalwert V_{rpm max} mindestens während eines Teils der Zeitdauer T, während der die Aortenklappe geöffnet ist, und
- optional Wiederholen der Schritte (a) bis (c) mindestens während der ventrikulären Systole eines nachfolgenden Herzzyklus.

2. Implantierbare Steuerungseinheit (14), **dadurch gekennzeichnet, dass** sie eine Energiequelle (16) und eine Zentraleinheit (17) mit einem Prozessor umfasst, wobei die Energiequelle (16) zur Energieversorgung einer Herzpumpe (13) bestimmt ist und wobei die Zentraleinheit (17) eine Gruppe von Softwarebefehlen umfasst, die bei Ausführung durch den Prozessor, die Durchführung eines Verfahrens zur Steuerung der Herzpumpe (13) erlauben, wobei die Herzpumpe (13) dazu bestimmt ist, Blut unter Druck mit einem Durchsatz zu fördern, der proportional zur Rotationsgeschwindigkeit Vᵣₚₘ der Pumpe (13) ist, wobei das Verfahren während einer gleichen ventrikulären Systole die folgenden Schritte umfasst:
a) Erfassen des Schließens der Mitralklappe des Herzens, wobei die Drehzahl Vᵣₚₘ der Pumpe (13) grundsätzlich kleiner als ein Maximalwert V_{rpm max} der Drehzahl der Pumpe (13) ist,
b) Erhöhen der Drehzahl Vᵣₚₘ der Pumpe (13), so dass zu einem Zeitpunkt t₂ nach dem Zeitpunkt t₁, der dem Schließen der Mitralklappe entspricht, die Drehzahl der Pumpe (13) gleich oder im Wesentlichen gleich dem Maximalwert V_{rpm max} der Drehzahl ist,
c) Halten der Drehzahl Vᵣₚₘ der Pumpe (13) auf diesem Maximalwert V_{rpm max} mindestens während eines Teils der Zeitdauer T, während der die Aortenklappe geöffnet ist, und
- optional Wiederholen der Schritte (a) bis (c) mindestens während der ventrikulären Systole eines nachfolgenden Herzzyklus.

3. Einheit nach Anspruch 2, **dadurch gekennzeichnet, dass** die Zentraleinheit (17) einen oder mehrere Eingänge zum Empfangen eines oder mehrerer Signale umfasst, von denen jedes mit einer hörbaren oder unhörbaren mechanischen Schwingung in Verbindung steht, die mit der mechanischen Aktivität des Herzens zusammenhängt, wobei die Zentraleinheit (17) eine ersten Untergruppe von Softwarebefehlen der Gruppe von Softwarebefehlen umfasst, die es bei Ausführung durch den Prozessor ermöglichen, ein Zeitfenster zum Messen des einen oder der mehreren Signale zu definieren, jedes so am Eingang der Zentraleinheit (17) während dieses Zeitfensters empfangene Signal zu analysieren, um einen oder mehrere Parameter des entsprechenden Signals zu bestimmen und den Parameter oder die Parameter jedes so bestimmten Signals mit einer oder mehreren zuvor in einer Speichereinheit der Zentraleinheit (17) aufgezeichneten Dateneinheiten zu vergleichen, um das dem Schließen der Mitralklappe entsprechende Signal sowie den dem Schließen der Mitralklappe entsprechenden Zeitpunkt t₁ zu identifizieren.

4. Zentraleinheit nach Anspruch 3, **dadurch gekennzeichnet, dass** die elektrische Herzaktivität mittels einer oder mehrerer Elektroden (18, 18') gemessen wird, wobei das oder die Messsignale an einem oder mehreren weiteren Eingängen der Zentraleinheit (17) empfangen werden, wobei die Zentraleinheit (17) eine zweite Untergruppe von Softwarebefehlen der Gruppe von Softwarebefehlen umfasst, die es bei Ausführung durch den Prozessor ermöglichen, in Echtzeit die Herzfrequenz des Herzens des Patienten zu bestimmen und die Herzpumpe (13) gemäß einem vorbestimmten Gesetz zu steuern, welches eine Funktion der so bestimmten Herzfrequenz ist, insbesondere ihrer Drehzahl Vᵣₚₘ.

5. Einheit nach Anspruch 4, **dadurch gekennzeichnet, dass** die Softwarebefehle der zweiten Untergruppe bei Ausführung durch den Prozessor ferner ermöglichen, aus einer Messung der elektrischen Herzaktivität jeden Zeitpunkt to zu bestimmen, zu dem eine ventrikuläre Depolarisation des Herzens auftritt, um die Schritte a) bis c) des Steuerungsverfahrens zu synchronisieren.

## Claims

1. Computer program comprising instructions suitable for implementing each of the steps of the method for managing a cardiac pump (13), when said program is run on a computer, said cardiac pump (13) being intended to send pressurized blood at a flow rate proportional to the speed of rotation Vᵣₚₘ of said pump (13), said method comprising, during a same ventricular systole, the following steps:
(a) detecting the closing of the mitral valve of said heart, the speed of rotation Vᵣₚₘ of said pump (13) being strictly less than a maximum value V_{rpm max} of the speed of rotation of said pump (13),
(b) increasing the speed of rotation Vᵣₚₘ of said pump (13) such that, at an instant t₂, after the instant t₁ corresponding to the closing of said mitral valve, the speed of rotation of said pump (13) is equal, or substantially equal, to said maximum value V_{rpm max} of the speed of rotation,
(c) keeping said speed of rotation Vᵣₚₘ of said pump (13) at this maximum value V_{rpm max} for at least a portion of the time period T during which the aortic valve is open, and
- optionally repeating the steps (a) to (c) for at least the ventricular systole of a next cardiac cycle.

2. Implantable management unit (14), **characterized in that** it comprises a power source (16) and a central unit (17) comprising a processor, said power source (16) being intended to power a cardiac pump (13), said central unit (17) comprising a set of software instructions which, when they are executed by said processor, make it possible to implement a method for managing said cardiac pump (13), said cardiac pump (13) being intended to send pressurized blood at a flow rate proportional to the speed of rotation Vᵣₚₘ of said pump (13), said method comprising, during a same ventricular systole, the following steps:
(a) detecting the closing of the mitral valve of said heart, the speed of rotation Vᵣₚₘ of said pump (13) being strictly less than a maximum value V_{rpm max} of the speed of rotation of said pump (13),
(b) increasing the speed of rotation Vᵣₚₘ of said pump (13) such that, at an instant t₂, after the instant t₁ corresponding to the closing of said mitral valve, the speed of rotation of said pump (13) is equal, or substantially equal, to said maximum value V_{rpm max} of the speed of rotation,
(c) keeping said speed of rotation Vᵣₚₘ of said pump (13) at this maximum value V_{rpm max} for at least a portion of the time period T for which the aortic valve is open, and optionally repeating the steps (a) to (c) for at least the ventricular systole of a next cardiac cycle.

3. Unit according to Claim 2, **characterized in that** said central unit (17) comprises one or more inputs for receiving one or more signals, each of which is linked to an audible or inaudible mechanical vibration linked to the mechanical activity of the heart, said central unit (17) comprising a first subset of software instructions of said set of software instructions which, when they are executed by said processor, make it possible to define a time window for measuring said signal or signals, for analyzing each signal thus received at the input of said central unit (17) during this time window to determine one or more parameters of the corresponding signal, for comparing the parameter or parameters of each signal thus determined with one or more data previously stored in a storage unit of said central unit (17) in order to identify the signal corresponding to the closing of the mitral valve and the instant t₁ corresponding to the closing of said mitral valve.

4. Unit according to Claim 3, **characterized in that** the electrical activity of the heart being measured by means of one or more electrodes (18, 18'), the measurement signal or signals being received at one or more other inputs of said central unit (17), said central unit (17) comprising a second subset of software instructions of said set of software instructions which, when they are executed by said processor, make it possible to determine in real time the heart rate of the heart of said patient, to monitor said cardiac pump (13) according to a predetermined law which is a function of said duly determined heart rate, in particular its speed of rotation Vᵣₚₘ.

5. Unit according to Claim 4, **characterized in that** said software instructions of said second subset also make it possible, when they are executed by said processor, to determine, from a measurement of the electrical activity of the heart, each instant to at which a depolarization a ventricular depolarization of said heart occurs in order to synchronize the steps a) to c) of said management method.
